# EUROPÄISCHE PATENTSCHRIFT

(11) **EP 1 684 868 B1**
(45) Veröffentlichungstag und Bekanntmachung des Hinweises auf die Patenterteilung: **02.11.2011**
(21) Anmeldenummer: 03816771.4
(22) Anmeldetag: 25.04.2003
(51) Int. Cl.: A61P 31/18, A61K 35/16, A61K 38/36, A61K 38/38

(54) **BEKÄMPFUNG VON HI-VIRUS INFEKTIONEN MIT DURCH HYPOCHLORIGE SÄURE OXIDIERTEM MENSCHLICHEM BLUTPLASMA**
TREATMENT OF HIV INFECTIONS WITH HYPOCHLOROUS ACID-OXIDISED HUMAN BLOOD PLASMA
LUTTE CONTRE DES INFECTIONS PAR LE VIH AU MOYEN DE PLASMA SANGUIN HUMAIN OXYDEES AVEC DE L'ACIDE HYPOCHLOREUX

(43) Veröffentlichungstag der Anmeldung: 02.08.2006
(73) Patentinhaber: Hamburger Stiftung zur Förderung von Wissenschaft und Kultur, 20354 Hamburg (DE)
(72) Erfinder: KEHREL, Beate, 48161 Münster (DE)
(74) Vertreter: Böhm, Brigitte
(86) Internationale Anmeldenummer: PCT/EP2003/004374
(87) Internationale Veröffentlichungsnummer: WO 2004/096368

(56) Entgegenhaltungen:
- WO-A-02/32445
- WO-A-91/04744
- DE-A- 10 045 047

## Beschreibung

Die Erfindung betrifft das Gebiet der Arzneimittel zum Bekämpfen einer Infektion einer Wirtszelle durch HI-Viren Zu diesen Zwecken werden erfindungsgemäß Arzneimittel ahgegeben, die oxidierte Proteine, und oxidierte Peptide enthalten, sowie Herstellverfahren. solcher Arzneimittel und therapeutische Anwendungsmöglichkeiten dieser Arzneimittel. Nachfolgend werden oxidierte Proteine und oxidierte Peptide gewonnen nach dem verfahren des Anspruchs 1 unter dem Begriff "oxP" zusammenfassend bezeichnet.

Wie in WO 02/22150 A2 und WO 02/32445 A2 beschrieben, binden oxP, wie, aber nicht beschränkt auf, "immune defense aktiviertes" Antithrombin (IDA-ATIII), oxidiertes Serumalbumin, oxidiertes Fibrinogen, an das GP120 des Env-Proteins in der Hülle des HI-Virus. OxPs sind in der Lage, wie in WO 02/22150 A2 beispielhaft von uns für IDA-ATIII gezeigt, die Vermehrung des HI-Virus in der Wirtszelle zu verhindern.

In Rahmen der vorliegenden Erfindung zeigen wir, daß oxPs in der Lage sind, den Kontakt zwischen dem HI-Virus und der Wirtszelle zu verhindern, die Bildung von Synzytien aus infizierten und nicht infizierten Abwehrzellen zu blockieren und damit die Infektion schon auf dem "Entry"-Level zu inhibieren. Überraschenderweise wurde zudem festgestellt, dass zum Unterbinden der Infektion einer Wirtszelle mit HI-Viren keine gereinigten oxidierten Proteine und oxidierten Peptide wie humanes Serumalbumin notwendig ist, sondern dass oxidiertes Blutplasma bereits als solches eine Infektion auf dem "Entry"-Level verhindern kann.

Obwohl einige potente antivirale Medikamente existieren, hat sich HIV zu einer echten weltweiten Pandemie entwickelt und ist somit die wichtigste Infektionskrankheit geworden. Über 4 Millionen Menschen versterben zur Zeit in jedem Jahr an dieser Erkrankung. Da heute zugelassene HIV-Medikamente wie Proteaseinhibitoren und reverse Transkriptaseinhibitoren nicht in der Lage sind, HIV von Infizierten Personen vollständig zu entfernen, besteht die dringliche Notwendigkeit neue antivirale Medikamente zu entdecken.

HIV-Infektionen / AIDS stellen eine der gewichtigsten Krisen in der Entwicklung der Menschheit dar. Daher muß ein HIV-Medikament gefunden werden, welches neben allen wissenschaftlichen Anforderungen, einerseits leicht herzustellen und erschwinglich ist und, andererseits für die Menschen speziell in den Sub-Sahara Gebieten leicht zugänglich gemacht werden kann.

Diese Probleme wurden mit der vorliegenden Erfindung gelöst, da die hier vorgestellten oxPs HIV-Infektionen schon auf der allerersten Eintrittsebene blockieren, oxPs leicht und billig herstellbar sind (es kann direkt Protein aus dem Plasma eines Menschen verwandt werden) und dieses Verfahren nicht nur in Speziallaboratorien durchgeführt werden kann.

Grundsätzlich dringen umhüllte Viren in ihre Target-Zelle ein, indem die Membran des Virus mit der Membran der Zielzelle in Kontakt tritt und anschließend beide Membranen so angenähert werden, daß sie fusionieren. Für den Kontakt des HIV-1 Virus mit der Wirtszelle ist die Bindung des extern liegenden HIV-Hüllenproteins Env an den Rezeptor CD4 erforderlich. Dies erfolgt über den Env-Bestandteil GP120. GP120 bindet nachfolgend an einen der Haupt-Korezeptoren CXCR4 oder CCR5. Die Anbindung von GP120 an seine Rezeptoren führt zu einer Konformationsänderung des externen GP120/GP41 Komplex. Diese Interaktion ist eine Grundvoraussetzung, die Bereitstellung des viralen GP41-N-Terminus zu ermöglichen, welches letzten Endes zur Membranfusion führt.

Ein Faktor, welcher den viralen Eintritt teilweise blockieren kann, sind neutralisierende Antikörper, die direkt gegen GP120 gerichtet sind. Jedoch besitzen neutralisierende Antikörper von natürlichen Infektionen nur begrerizte Effektivität, da zum einen HIV eine sehr große Vielzahl von verschiedenen Antigen-Varianten produziert, es zum anderen nach einer Infektion zu einer einschränkenden *"klonalen Dominanz"* von HIVneutralisierenden Antikörpern kommt. Daher können neue HIV-1 Varianten leicht solch einer hoch spezifischen aber eingeschränkten Antwort entkommen.

Jüngste Erkenntnisse haben gezeigt, daß die nicht-spezifische, angeborene Immunantwort relevant für die Verteidigung gegen HIV ist. Polymorphkernige neutrophile Leukozyten (PMNL) und Monozyten sind, nach Stimulation, virizidal gegenüber HIV-1. Lipopolysaccharid (LPS) - Stimulation, welche zum oxidativen Burst von Leukozyten führt, bewirkt eine Blockade des HIV-Eintritts, ohne Berücksichtigung des viralen Korezeptorphänotyps.

Leukozyten generieren H₂O₂ und sezernieren das Haem-Protein Myeloperoxidase (MPO). Klebanoff und seine Mitarbeiter haben gezeigt, daß stimulierte PMNL von Patienten mit vererbter Defizienz des Enzyms MPO eine verminderte virizidale Aktivität besaßen. Durch Zusatz von MPO konnte die verminderte Abwehrkraft wieder rekonstituiert werden. Basierend auf dem gegenwärtigen Erkenntnisstand wird angenommen, daß das MPO Produkt HOCI selbst das antiviral agierende Agens ist. Die Expression und die Freisetzung der MPO, welche HOCI produziert, ist *in vivo* streng kontrolliert. Freies HOCI stellt einen Teil des oxidativen Stresses dar.

Wir haben nun im Rahmen der Erfindung festgestellt, daß Proteines und Peptide, welche in Kontakt mit HOCl gekommen sind, in eine antivirale Form transformiert werden kann und dadurch HOCI neben der bekannten direkten Wirkung eine indirekte Wirkung gegen HIV-1 hat.

Zur Relevanz dieser Art der Inhibition für zukünftige therapeutisch Applikationen: HOCl, welches von der MPO in entzündetem Gewebe produziert wird, modifiziert LDL und viel andere humane Proteine *in vivo.* HOCI-modifizierte Proteine könne durch spezifische monoklonale Antikörper ( WO-02/32445 A2) detektiert werden. Daher bietet eine strukturelle Veränderung, die durch HOCI Behandlung generiert wird, ein Epitop an, welches schon *in vivo* vorhanden ist und daher dem Immunsystem gut bekannt ist. Daher sollten oxPs *in vivo* toleriert werden.

Zusätzlich beruht oxP auf einer relativ einfachen und mit geringen Kosten verbundenen chemischen Modifikation.

Warum produziert der Körper nicht genug oxP *in vivo,* um sich vor HIV Infektion zu schützen? Eine Antwort auf diese Frage könnte die Beobachtung sein, daß sich die Funktion der neutrophilen Leukozyten und der Monozyten bei HIV-infizierten Personen zu Beginn der Infektion verschlechtert und daß die Funktionsverluste positiv korrelieren mit dem Ausmaß/Fortschreiten der HIV induzierten Erkrankung

Die Aufgabe der vorliegenden Erfindung war es daher, ein Arzneimittel bereitzustellen, welches eine HIV-Infektion schon auf dem "Entry"-Level inhibiert, und gleichzeitig einfach und preisgünstig herzustellen und damit auch für Patienten in der s.g. "Dritten Welt" anwendbar ist.

Die Aufgabe wird gelöst durch ein Herstellverfahren für ein Arzneimittel zum Bekämpfen einer Infektion einer Wirtszelle durch HI-Viren das gekennzeichnet ist durch die Schritte:
a) Bereitstellen von menschlichem Bentplasma
b) Oxidieren der im Gemisch enthaltenen Proteine und Peptide, mit Hoce.

Während in früheren Untersuchungen die Wirkung einzelner, gereinigter Proteine auf die Bindung eines Env-Proteins an ein CD4-Protein gezeigt wurde, hat sich nunmehr überraschenderweise herausgestellt, dass die Infektion einer Wirtszelle durch HI-Viren auch durch Proteingemische gehemmt, vermindert oder sogar vollständig unterbunden werden kann. Insbesondere können diese Wirkungen auch durch Komplexe protein- und/oder peptidhaltige Gemische mit mehr als einer Protein- bzw. Peptidsorte erreicht werden. Dies war überraschend, da zu erwarten war, dass der Prozess der Herstellung der zum Bekämpfen einer Infektion einer Wirtszelle durch HI-Viren und/oder zum Hemmen der Bindung eines Env-Proteins an ein CD4-Protein notwendigen oxidierten Proteine und/oder Peptide bei der Oxidation in solchen komplexen Gemische durch Erzielung falscher Konformatioen und durch Nebenreaktionen derart beeinträchtigt wird, daß die Wirksamkeit von oxidierten Proteinen in solchen Gemischen herabgesetzt oder völlig aufgehoben ist.

Im Sinne dieser Erfindung ist Vollblut das einem Menschen

entnommene und gegebenenfalls mit einem geeigneten Antikoagularis vermischte Blut.

Im Rahmen der vorliegenden Erfindung ist, wie oben bereits ausgeführt, festgestellt worden, daß oxP die Bindung von HIV-GP120 an CD4 blockiert und somit das Eindringen des HIV in die Zielzelle verhindert. Da ein Virus für seine Vermehrung aber auf das Eindringen in eine Wirtszelle angewiesen ist, kann er sich nicht weiter vermehren. Die Synzytienbildung aus HIV- infizierten Abwehrzellen mit nicht infizierten Abwehrzellen unterbleibt, so daß diese ihre Aufgaben weiter wahrnehmen, und infizierte Zellen vernichten können.

Plasmaproteine und Plasmapeptide sind solche Proteine bzw. Peptide, die in der Flüssigkeit enthalten sind, die sich bei Zentrifugieren oder Sedimentieren von Vollblut oben absetzt, und die aus ihnen herstellbaren Proteine, Peptide und Protein- bzw. Peptidkomplexe. Zu den Plasmaproteinen bzw. Plasmapeptiden zählen insbesondere Serumalbumin, insbesondere humanes Serumalbumin und Rinderserumalbumin, Antithrombin, "immune defense aktiviertes" Antithrombin (IDA-ATIII), Fibrinogen, Gerinnungsfaktoren und Immunglobuline.

Das verfahrensgemäß hergestellte Arzneimittel enthält also oxidierte Proteine, oxidierte Peptide, und oxidierte Aminosäuren, (zusammengefasst als oxP).

Erfindungsgemäß können Plasmafraktionen (Plasmaproteingemische) des Patienten selbst oder eines Spenders ohne notwendige Isolierung der Proteine, direkt in ein Medikament verwandelt werden, welches oxP enthält.

Ein erfindungsgemäßes Arzneimittel kann selbstverständlich weitere pharmazeutisch annehmbare Hilfs- oder/und Trägersubstanzen enthalten, wobei das Arzneimittel zur lokalen, intradermalen, oberflächlichen, intraperitonalen, intravenösen, intramuskulären oder oralen Verabreichung formuliert wird oder seine Verabreichung über Vesikel ermöglicht. Das erfindungsgemäße Arzneimittel enthält daher vorzugsweise solche Hilfs- und Trägersubstanzen, welche die jeweilige bevorzugte Applikationsart ermöglicht.

Das erfindungsgemäße Arzneimittel kann selbstverständlich neben oxP weitere Substanzen enthalten, wie beispielsweise Antibiotika, andere HIV-Infektionshemmer etc. Es kann je nach zu behandelnder Begleitkrankheit von Vorteil sein, unterstützend mit bekannten Arzneimitteln zu behandeln. Eine entsprechende Kombination dieses Arzneimittels mit oxP ist daher gegebenenfalls eine bevorzugte Ausführungsform der vorliegenden Erfindung.

Die Erfindung wird nachfolgend anhand der Beispiele und der Figuren näher beschrieben.

1.) Beispiel für die Herstellung von oxidiertem HSA gemäß Anmeldung WO 02/32445 A2 (Vergleichsbeispiel)

Um normales Humanserumalbumin in die antivirale Form zu transformieren, wurde HSA mit HOCl aktiviert. Frisch hergestelltes HOCI wurde in einem molaren Verhältnis von 1:100 zu HSA gegeben. Nach einer Inkubationszeit von 30 Minuten bei Raumtemperatur wurde das verbleibende Hypochlorit durch Gelfiltration (Sephadex G25) entfernt.

In einer anderen Anwendung wurden mit einem Standardverfahren (z.B. Ammoniumsulfatfällung/Entsalzung oder Kryopräzipitation)

Proteingemische aus humanem Plasma isoliert und diese dann direkt mit frisch hergestelltem HOCI , wie für Humanalbumin beschrieben, modifiziert.

### Oxidieres HSA 2.) ist nicht zytotoxisch. (Vergleichsbeispiel)

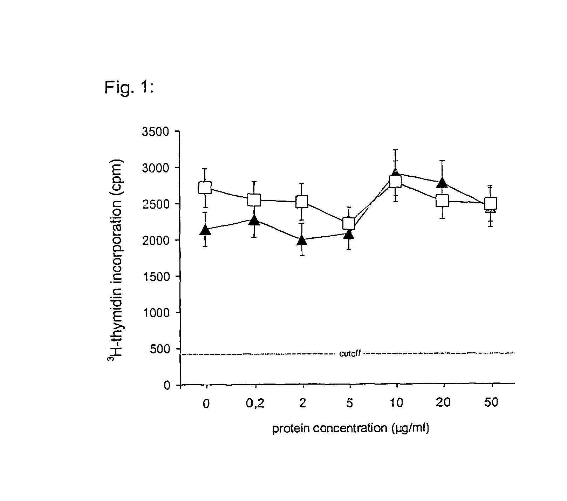

Das HOCI modifizierte HSA wurde zuerst im ³H-Tymidin-Inkorporations Assay getestet. Bis zu einer Gebrauchskonzentration von 50µg/ml konnte keine anti-zelluläre Aktivität in Hinblick auf die Zellproliferation von Hela oder GHOST Zellen im Vergleich zu normalem HSA beobachtet werden (Abb.1).

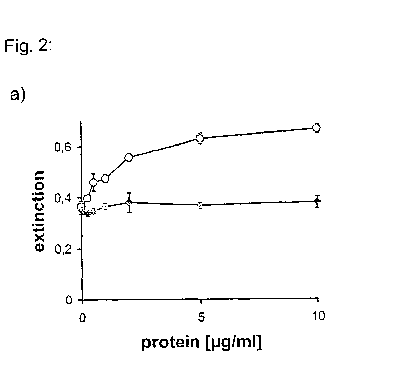

### 3.) (Vergleichsbeispiel) Die Anbindung von oxidiertem HSA an IIIB GP120 (aus dem AIDS Reagenz Projekt EVA) wurde in einem Standard-ELISA Assay veranschaulicht (Abb. 2a).

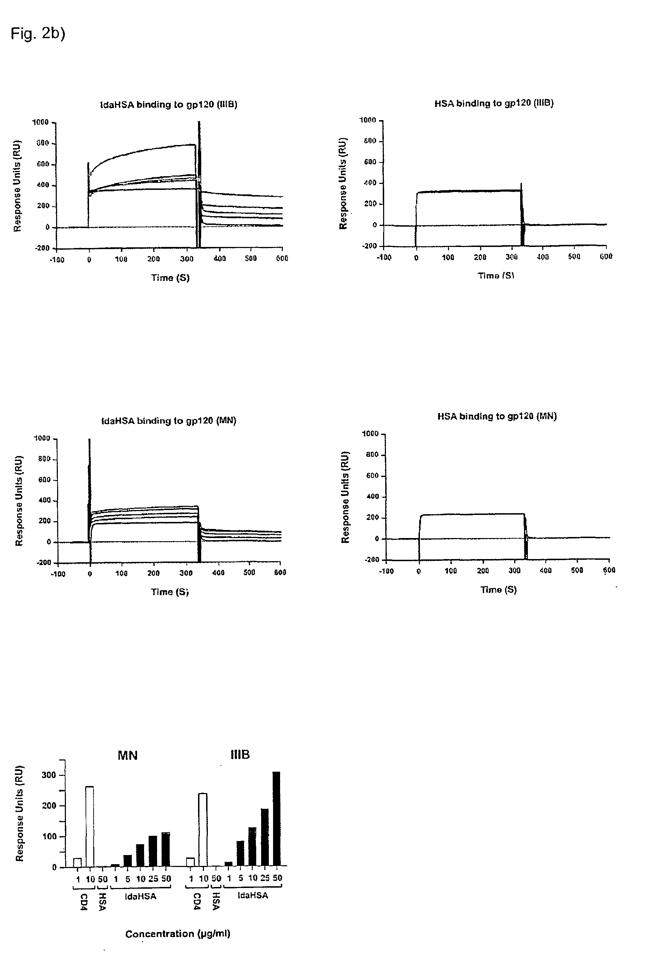

Zusätzlich wurde die Bindung von oxidiertem HSA sowohl an IIIB GP120, als auch an SF2 GP120 in der Oberflächen Plasmon Resonanz Spektroskopie (SPR) gezeigt (Abb. 2b). In beiden Versuchen wurde die direkte Interaktion von Oxidiertem HSA an GP120 gezeigt Nur nach Transformation des Proteins in die oxidierte Form konnte eine spezifische Bindung beobachtet werden. Bei Gebrauch von normalem Protein, hier HSA, normalem Rinderserumalbumin, Glutathion-S-Transferase (GST) oder mit einem GST-V3 Fusionsprotein, welches das FP120 V3 Loop beinhaltete konnte keine Bindung beobachtet werden. In all diesen Kontrollversuchen waren die "Response Units" (RU) <5. Zusätzlich zur SPR Anbindungsuntersuchung wurde die Kinetik der oxP (hier ox ATIII) GP120-IIIB Interaktion untersucht. Die Analyse ergab ka und kd Werte von 1,47 *10⁻⁹ und 7,01* 10⁻¹⁰ M, daraus resultierend eine KD von 7,0 *10⁻¹⁰ M (Rmax=120; Chi2=40).

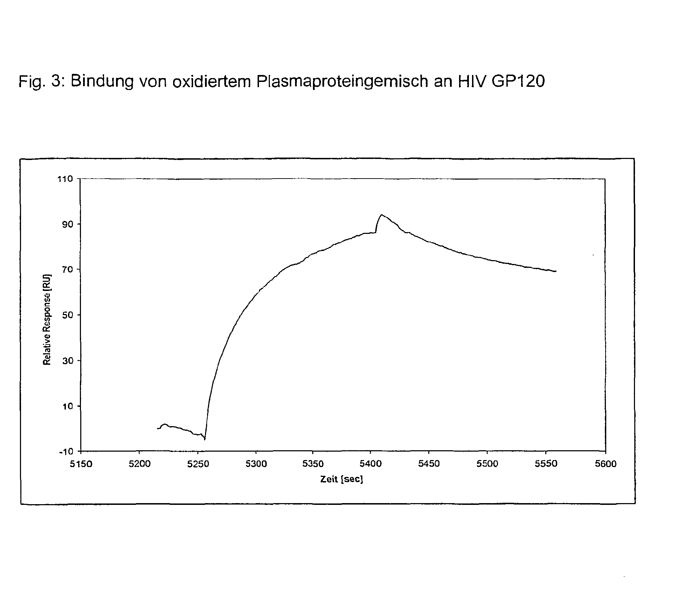

### 4.) Erfindungsgemäß Ein unfraktioniertes Proteingemisch aus menschlichem Plasma bindet nach Behandlung mit HOCl, wie oben beschrieben, an HIV-GP120. (Abb. 3)

### 5.) Oxidiertes HSA neutralisiert HIV (Vergleichsbeispiel)

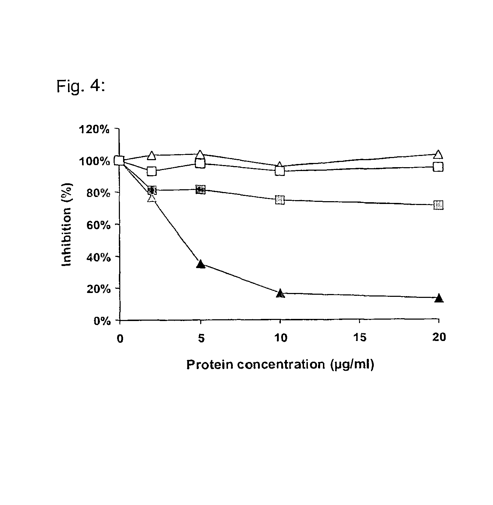

Für HIV Neutraliastions-Experimente wurden die HIV-1 Stämme NL4-3 und eine Variante des NL4-3, NL-991, verwandt, in welchem der V3 Loop gegen ein V3 Loop vom Primärisolat PI-991 ausgetauscht wurde. NL4-3 ist ein monotropes Virus, weiches nur den CXCR4 Ko-Rezeptor verwendet. Der NL-991 Virus ist R5-monotrop und gebraucht nur CCR5 als Ko-Rezeptor. In Abb. 4 wird gezeigt, daß die Replikation beider Viren durch oxPHSA inhibiert wird. Dies spiegelt sich in der Menge des produzierten HIV p24-Antigens wider.

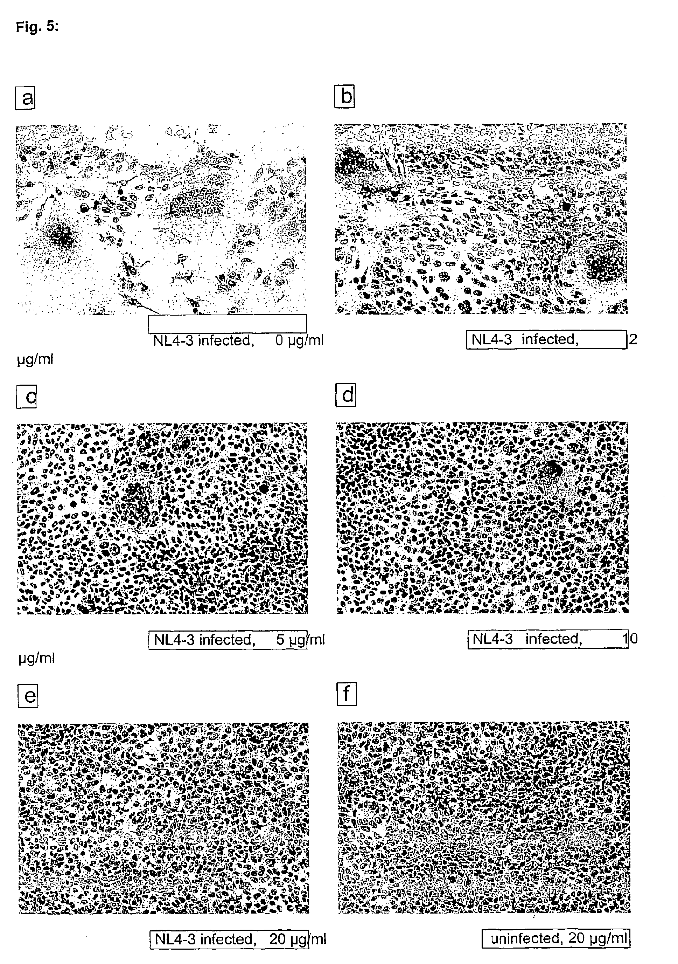

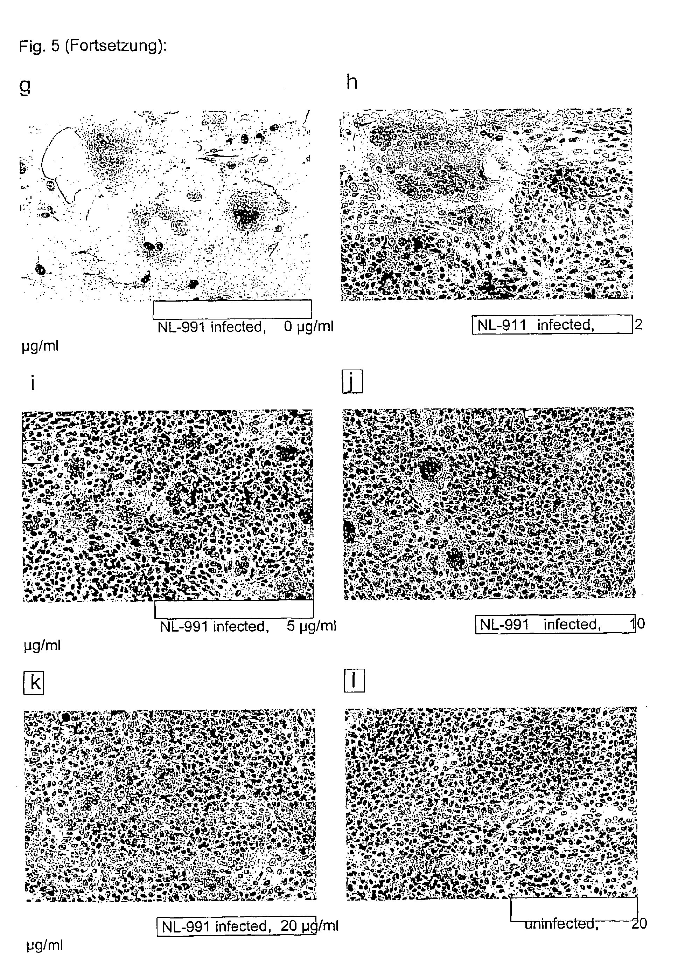

6.) Vergleichsbiespiel in HIV-Zellkulturen fusionieren HIV infizierte Zellen mit nicht- infizierten CD4⁺-Targetzellen. Diese Fusion unter den Zellen ist bekannt als Synzytien Bildung. Diese Synzytien Bildung ist auf die Anbindung von GP120, welches auf der Membran infizierter Zellen exprimiert wird, an den CD4 Rezeptor auf der Targetzelle und anschließende Insertion des GP41 N-Terminus in die Target-Membran zurückzuführen. Beide Viren, die in diesem Neutralisations-Assay verwandt wurden (NL4-3 und NL-991) waren in der Lage, Synzytien mit den GHOST-CXCR4 und GHOST-CCR5 Zellen zu bilden (Abb. 5). Sowohl die Synzytien Bildung induziert durch den NL4-3 Virus (Abb. 5a), als auch durch den NL-991 Virus (Abb 5g), konnte durch Zusatz von ox-HS4 in Konzentrationen bis 20µg/ml inhibiert werden (Abb. 5e-4k). oxHSA zeigte eine dosisabhängige Inhibition (5b-e; 5h-k). Wie schon in Abb. 1 gezeigt, beeinflußte oxHSA selhst weder die Zellproliferation, noch die Zellmorphologie (Abb. 5f; 51) und die Anfärbung der Zellkerne bewies die Vitalität der GHOST Zellen.

### 7.) OxHSA blockt die HIV-Infektion auf dem "Entry-Level" (Vergleichsbeipiel)

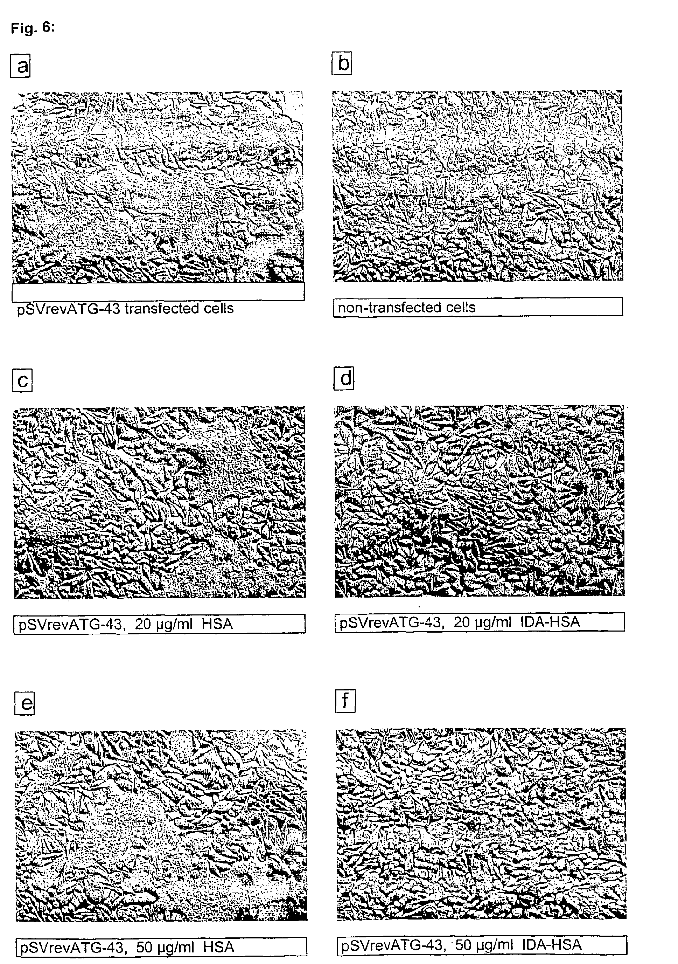

Die Synzytien Bildung basiert auf der Präsenz der viralen Hülle und der viralen Andockproteine auf der Membranoberfläche der Wirtszellen. Deshalb wurden Hela-P4 Zellen verwandt (CD4+, CXCR4+, CCR5+), welche zusätzlich die viralen Rezeptoren GP120/GP41 exprimierten. Hela-P4 Zellen wurden dazu mit GP160 Vektoren transfiziert, so daß sie die Env-Proteine von HIV- NL4-3 und HIV-NL-911 exprimierten. Gp160 transfizierte Hela-P4 Zellen fusionierten und bildeten nach 28 h im Gegensatz zu nicht-transfizierten Zellen (6b; 6h) Synzytien aus (Abb. 6a; 6g).

Die Inkubation mit oxHSA führte zu einer dosisabhängigen Inhibierung der Bildung von Synzytien (6d, f, 6j, I) im Gegensatz zur Inkubation mit nicht modifiziertem Protein (6c, e, i, k). Dieser Transfizierungs-Assay ahmt den Eintritt von X4- und R5-tropen HI-Viren (NL4-3, NL-911) nach. In beiden Synzytientestverfahren zeigte oxHSA anti-HIV-entry" Aktivität bei 20 und 50µg/ml. Dies veranschaulicht, daß oxHSA auf dem GP120-CD4 Interaktions Level mit einer ID>95 bei 50µg/ml-agiert.

### 8.) Fällung von Plasmaproteinen und deren Oxidation (Erfindungsgemäß)

10 ml Citratblut wurden bei 3200 rpm / 2000g für 10 Minuten zentrifugiert und das überstehende Plasma abgenommen. In einem Volumenverhältnis von 1:1 wurde 5 M (NH4)2SO4 zugesetzt, das Plasma 20 Minuten gerührt und dabei die Plasmaproteine ausgefällt. Die Suspension wurde emeut für 10 Minuten bei 3200 rpm/2000g zentrifugiert, der Überstand verworfen und die gefällten Proteine in PBS Puffer (pH 7,4) resuspendiert. Die Lösung wurde in einen Dialyseschlauch gefüllt (Ausschlußgrenze, 10.000 D) und für 3 Tage gegen PBS-Puffer dialysiert. Dabei wurde der Puffer 3 mal gewechselt. Alternativ wurde das Proteingemisch durch ein Gelfiltrationsverfahren entsalzt.

Nach der Dialyse/ Gelfiltration wurde der Gesamtproteingehalt photometrisch durch Standardverfahren bestimmt.

Oxidation zum erfindungsgemäßen Arzneimittel:
Zu 500µg der Proteinlösung wurde frisches HOCl (12 µl) gegeben und mit PBS/0,1mM EDTA-Puffer auf ein Volumen von 1 ml aufgefüllt. Nach 15 Minuten Reaktionszeit auf Eis ( 0°C) wurde die Lösung auf eine mit PBS-Puffer äquilibrierte Gelfiltationssäule gegeben und so das nicht umgesetzte HOCl entfernt.
Fig. 1: IDA (immune defense obtinertes)-HSA ist nicht cytotoxisch
Hela Zellen, die humanes CD4, CXCR4 und CCR5 exprimierten, wurden in der Anwesenheit von [³H]-Tymidin und unterschiedlichen Konzentrationen von IDA-HSA (□) oder HSA (▲) kultiviert. Die Zellen wurden zu 10⁴ Zellen pro "Well" in einer 96 "Well"-Platte dreifach ausgesät. An Tag 2 wurde zu jedem "Well" [³H]-Tymidin (10 µCi/ml) gegeben. Nach 8 Stunden wurde die DNA geerntet, an eine Glasfiber Membran gebunden und eingebautes [³H]-Tymidin mit einem β-Counter quantifiziert.
Fig. 2: IDA-HSA bindet an HIV-1 GP120
Die spezifische Anbindung von IDA-HSA an GP120 wurde in einem Standard-ELISA Verfahren (a) und durch Oberflächen Plasmon Resonanz Spektroskopie (SPR) (b) veranschaulicht. Für den ELISA wurde eine 96-Well-Platte mit 100µl/well rekombinantem GP120 (1µg/ml) gecoatet und anschließend mit 0,25% Gelatine (in PBS) geblockt (1h RT). IDA-HSA (○) und HSA (●) wurden in unterschiedlichen Konzentrationen aufgegeben (0, 0.25, 0.5, 1, 2, 5, 10, 20µg/ml in PBS). Gebundenes Protein wurde mit HRP-konjugierten, polyklonalen, spezifischen anti-HSA Antikörpern (Sigma) detektiert. Für die SPR wurde GP120 (10µg/ml) kovalent mit EDC/NHC an einen Dextran gecoateten, CH-aktivierten Sensor-Chip (CM5, Biacore, Sweden) gebunden. Die Durchflußrate betrug für 10 Minuten 5µl/min. Nach dem Blocken mit Ethanolamin wurde die Anbindung von IDA-HSA und HSA (je 1µg/ml) bei einer Flußrate von 5µl/min für 6 Minuten untersucht.
Fig. 3: Oxidierte Plasmaproteingemische binden an HIV-GP120
Die spezifische Anbindung von einem Gemisch oxidierter Plasmaproteine an GP120 wurde durch Oberflächen Plasmon Resonanz Spektroskopie (SPR) gezeigt. GP120 (10µg/ml) wurde an einen Sensor-Chip kovalent (C1, Biacore, Sweden) gebunden.

| | |
|---|---|
| Injektion von 20 µl 100 mM Glycin, 0,3 % Triton X-100 pH 12 (zweimal) | Reinigung der Sensoroberflä che |
| 5 µl/min | Flußrate |
| Injektion von 20 µl 400 mM EDTA | Entfernen von Calcium-Ionen von der Sensoroberflä che |
| Injektion von 50 µl NHS/EDC | Aktivierung der Sensoroberflä che |
| Injektion von 20 µl 100 nM P120 In 10 mM NaAc pH 4 (Verdünnung 1:10) | Kovalente Kopplung von P120 |
| Injektion von 55 µl Ethanolamin | Blocken der verbleibenden aktivierten Ester |

Anschließend wurde eine Lösung von 93,5 nM PluO über immobilisiertes P120 injiziert. Es wurde eine eindeutige Bindungskurve (s.o.) erhalten. In nachfolgenden Experimenten wurde gezeigt, daß die resultierende Signalhöhe mit der Menge an immobilisiertern P120 korreliert.
Fig. 4: Inhibierung der HIV-Replikation
GHOST-CXCR4 oder GHOST-CCR5 Zellen wurden mit X4-tropen NL4-3 (Dreiecke) oder R5-tropen NL-991 Viren (Quadrate) (500TCID₅₀) infiziert. Zellkultur-Überstand wurde an Tag 5 mit einem p24 Standard-ELISA auf p24-Antigen getestet. Gezeigt ist der Mittelwert von 3 Messungen. Der Standardfehler für den Mittelwert war <10%, NL4-3 + IDA-HSA (▲); NL4-3 + HSA (△); NL-991 + IDA-HSA (■); NL-991 + HSA (□).
**Fig. 5****: Inhibierung der HIV-Induzierten Synzytien Bildung**
GHOST-CXCR4 öder GHOST-CCR5 Zellen wurden mit 500 TCID₅₀ der HIV-Laborisolate (A-E) NL4-3 (X4-monotrop) oder (F-L) NL-991 (R5-monotrop) infiziert. Die Infektion wurde durch Zugabe von IDA-HSA Protein mit einer Endkonzentration von 0, 2, 5, 10 oder 20µg/ml zum Kulturmediums inhibiert. Die Infektion wurde 5 Tage nach Infektionsbeginn durch Darstellung der induzierten Synzytien und der Zerstörung des Zellrasens sichtbar gemacht. Dazu wurden die Zellen/Kerne durch eine Standard Eosin/Methylen blau/ Azur Färbeprozedur (Hemacolor, Merck) angefärbt. (a), NL4-3 infizierte Zellen; (b) NL4-3 + 2µg/ml IDA-HSA; (c) NL4-3 + 5µg/ml IDA-HSA; (d) NL4-3 + 10µg/ml IDA-HSA; (e) NL4-3 + 20µg/ml IDA-HSA; (g) NL-99.1 infizierte Zellen; (h) NL-991 + 2µg/ml IDA-HSA; (i) NL-911 + 5µg/ml IDA-HSA; (j) NL-911 + 10µg/ml IDA-HSA; (k) NL-91.1 + 20µg/ml IDA-HSA; (l) 20µg/ml IDA-HSA.
Fig. 6: Inhibierung der GP160-induzierten Synzytien Bildung
Hela Zellen, welche humanes CD4, CXCR4 und CCR5 exprimieren, wurden mit pSVATGrev Plasmiden transfiziert, die NL4-3 oder NL-911 env exprimieren. Dazu wurde entweder IDA-HSA, oder HSA Protein gegeben (Endkonzentration 20 und 50µg/ml). Die Synzytien Bildung wurde nach 28 Stunden durch Standard Phasen Kontrast Mikroskopie der lebenden Zellen untersucht. IDA-HSA verhinderte die Synzytienbildung in dosisabhängiger Weise.

### Referenzen:

Stephenson J: Growing, Evolving HIV/AIDS Pandemic Is Producing Social and Economic Fallout JAMA. 2003;289:31-33.
2 Brenner BG, Tumer D, Wainberg MA: HIV-1 drug resistance: can we overcome? Expert Opin Biol Ther. 2002;2(7):751-61.
3 Kwong PD, Wyatt R, Robinson J, Sweet RW, Sodroski J, Hendrickson WA. Structure of an HIV gp120 envelope glycoprotein in complex with the CD4 receptor and a neutralizing human antibody. Nature. 1998;393(6686):648-59
4 Gaschen B, Taylor J, Yusim K, Foley B, Gao F, Lang D, Novitsky V, Haynes B, Hahn BH, Bhattacharya T, Korber B. Diversity considerations in HIV-1 vaccine selection. 'Science. 2002;296(5577):2354-60.
5 Muller S, Wang H, Silverman GJ, Bramlet G, Haigwood N, Kohler H. B-cell abnormalities in AIDS: stable and clonally-restricted antibody response in HIV-1 infection. Scand J Immunol. 1993;38(4):327-34.
6 Nara PL, Garrity RR, Goudsmit J. Neutralization of HIV-1: a paradox of humoral proportions. FASEB J. 1991 5(10):2437-55.
7 Verani A, Sironi F, Siccardi AG, Lusso P, Vercelli D. Inhibition of CXCR4-tröpic HIV-1 infection by lipopolysaccharide: evidence of different mechanisms in macrophages and T lymphocytes. J Immunol. 2002;168(12):6388-95.
8 Chase MJ, Klebanoff SJ Viricidal effect of stimulated human mononuclear phagocytes on human immunodeficiency virus type 1. Proc Natl Acad Sci USA. 1992 ,15;89(12):5582-5.
9 Klebanoff SJ, Coombs RW. Viricidal effect of polymorphonuclear leukocytes on human immunodeficiency virus-1. Role of the myeloperoxidase system. J Clin Invest. 1992;89(6):2014-7.
10 Polzer S, Dittmar MT, Schmitz H, Schreiber M. The N-linked glycan g15 within the V3 loop of the HIV-1 external glycoprotein gp120 affects coreceptor usage, cellular tropism, and neutralization. Virology. 2002 ,5;304(1):70-80.
11 Daugherty A, Dunn JL, Rateri DL, Heinecke JW. Myeloperoxidase, a catalyst for lipoprotein oxidation, is expressed in human atherosclerotic lesions. J Clin Invest. 1994;94(1):437-44.
12 Sugiyama S, Okada Y, Sukhova GK, Virmani R, Heinecke JW, Libby P. Macrophage myeloperoxidase regulation by granulocyte macrophage colony-stimulating factor In human atherosclerosis and implications in acute coronary syndromes. Am J Pathol. 2001;158(3):879-91
13 Spada C, Treitinger A, Fujimura AY. Morphofunctional Study of Blood Polymorphonuclear Leucocytes in HIV-Seropositive Individuals. Braz J Infect Dis. 1998;2(6):285-290
14 Karen C. Hayani, Stephen C. Verral, and David L. Pitrak Impaired Phagocyte Oxidative Capacity in Human Immunodeficiency VirusInfected The Journal of Infectious Diseases 1999;179:584-589
15 WO-A-02/22150
16 WO-A-02/32445

## Patentansprüche

1. Verfahren zum Herstellen eines Arzneimittels zum Bekämpfen einer Infektion einer Wirtszelle durch HI-Viren,
**gekennzeichnet durch** die Schritte:
a) Bereitstellen von menschlichem Blutplasma und
b) Oxidieren der im Blutplasma enthaltenen Proteine und Peptide mit HOCl.

2. Arzneimittel zur Verwendung zum Bekämpfen einer Infektion von Wirtszellen durch HI-Viren, herstellbar nach Anspruch 1.

## Claims

1. A method for producing a medicament for combating an infection of a host cell by HI viruses,
**characterised by** the steps:
a) providing human blood plasma and
b) oxidising the proteins and peptides present in the blood plasma with HOCl.

2. A medicament, producible according to claim 1, for use in combating an infection of host cells by HI viruses.

## Revendications

1. Procédé pour produire un médicament pour combattre une infection d'une cellule hôte par des virus IH **caractérisé par** les étapes :
a) fourniture de plasma sanguin humain et
b) oxydation des protéines et peptides contenus dans le plasma sanguin avec HOCl.

2. Médicament destiné à être utilisé pour combattre une infection de cellules hôtes par des virus IH pouvant être produit selon la revendication 1.
